# EUROPEAN PATENT SPECIFICATION

(11) **EP 2 441 377 B1**
(45) Date of publication and mention of the grant of the patent: **28.06.2017**
(21) Application number: 11184719.0
(22) Date of filing: 11.10.2011
(51) Int. Cl.: A61B 1/00

(54) **SWITCHING VALVE ASSEMBLY FOR ENDOSCOPE**
Schaltventilanordnung für Endoskop
Ensemble formant soupape de commutation pour endoscope

(30) Priority: 12.10.2010 JP 2010229700
(43) Date of publication of application: 18.04.2012
(73) Proprietor: Fujifilm Corporation, Minato-ku Tokyo (JP)
(72) Inventor: Morimoto, Yasuhiko, Kanagawa (JP)
(74) Representative: Klunker IP Patentanwälte PartG mbB

(56) References cited:
- DE-A1-102004 003 857
- JP-A- 7 008 449
- JP-A- 8 238 212
- JP-A- 2006 175 175
- JP-A- 2009 045 126
- US-A- 4 261 343
- US-A- 4 800 869
- US-A- 5 840 016
- US-A- 5 871 441
- US-A1- 2010 049 001

## Description

### BACKGROUND OF THE INVENTION

### 1. Field of the Invention

The present invention relates to a switching valve assembly for endoscope. More particularly, the present invention relates to a switching valve assembly for endoscope, in which depression stroke of a piston unit can be reduced, and in which flow lines can be changed over reliably by depressing operation of the piston unit.

### 2. Description Related to the Prior Art

Ultrasonic examination is known in the medical field. Ultrasonic waves are applied to a body, from which reflected waves or echo is received to form an image of an object in the body. An example of the ultrasonic examination is an endoscopic examination, in which an instrument with ultrasonic transducers is entered in a body cavity and emits the ultrasonic waves. The endoscopic examination is advantageous in comparison with examination with the ultrasonic waves percutaneously, because a condition of tissue of a stomach, large intestine or other organs can be imaged very precisely. Importance of the endoscopic examination is typically great for precise diagnosis of a tumor, ulcer and the like of a body cavity, in relation to their depth and other attributes.

For the endoscopic examination, an ultrasonic endoscope or an ultrasonic probe is used. The ultrasonic endoscope has an array of ultrasonic transducers, CCD as an image sensor and the like incorporated in a head assembly. The ultrasonic probe is used by entry in an instrument channel of an endoscope. In the field of ultrasonic imaging, there is a serious problem of attenuation of the ultrasonic waves in the presence of air for application of the ultrasonic waves from the tip of the ultrasonic endoscope or the ultrasonic probe to a wall of a body cavity. In view of this problem, a balloon is disposed at the tip of the ultrasonic endoscope or the ultrasonic probe for covering the ultrasonic transducers. The balloon is charged with water as a transmission medium and expanded, and then set in tight contact with the wall of the body cavity. Then the ultrasonic waves are emitted from the inside of the balloon. Thus, attenuation of the ultrasonic waves with air is prevented. When the endoscopic examination is completed, water is drained from the balloon to compress the balloon, before the ultrasonic endoscope is removed easily from the body.

An air/water supply button assembly and a suction button assembly are disposed on a handle device of the ultrasonic endoscope. Expansion and compression of the balloon are changed over by pushing the air/water supply button assembly and the suction button assembly. In general, a two-step button is used as an example of each of the air/water supply button assembly and the suction button assembly. Document US4261343 discloses a switching valve assembly according to the preamble of independent claim 1.

Document JP2009-45126 discloses a 2 stage switching valve assembly. Document DE102004003857 discloses a switching valve assembly, provided with a sealing between the valve button and the cylinder/piston; it also discloses the provision of vent holes in the button body; the sealing in combination with the vent holes results in a better / stronger suction. JP-A 10-028670 (corresponding to JP-B 3017957) and JP-A 2007-111266 disclose the air/water supply button assembly, which includes a button device or top cap device, and a vent formed in the button device. When an air supply pump is driven without pushing the air/water supply button assembly, air is leaked through the vent in the button device. When the vent is closed, a check valve is opened in the air/water supply button assembly, so that an air/water supply nozzle ejects air, because the vent is closed in the air supply state. When the button device is depressed halfway, the operation is changed over from the air supply to the water supply. The air/water supply nozzle ejects water. When the button device is depressed fully, the water channel is changed over. A balloon channel supplied water to the balloon for expansion.

A suction pump operates for suction of air. In an inactive condition of the suction button assembly according to JP-A 10-028670 and JP-A 2007-111266, the suction pump draws external air through a vent channel. When the button device of the suction button assembly is depressed halfway, an instrument channel comes to communicate with the suction pump, and operates for the suction. When the button device is depressed fully, the suction pump comes to communicate with the balloon channel. Water is drained from the balloon through the balloon channel to compress the balloon.

The suction button assembly as two-step button includes a cylinder, a piston, the button device, and a housing wall. The piston is contained in the cylinder in a slidable manner. There is an end opening of a cylinder passage formed in the cylinder, at which an upper end of the piston protrudes. The button device is formed with the upper end of the piston. The housing wall is disposed to cover the end opening, and keeps the button device operable for depression to a halfway position and a down position.

Plural channels are connected to the cylinder passage, including a discharge conduit, a suction channel and a balloon compression channel. The discharge conduit extends to the suction pump. The suction channel extends to the instrument channel. The balloon compression channel extends to the balloon channel. A valve head or piston rod or lower stem end is a lower end of the piston. A flow channel is formed through the valve head to extend between a side wall and a lower end point of the valve head. Also, a groove is formed in the side wall of the valve head. When the button device is depressed halfway, the flow channel and the groove operate for the discharge conduit to communicate with the suction channel. When the button device is depressed fully, the flow channel and the groove operate for the discharge conduit to communicate with the balloon compression channel. To this end, the flow channel and the groove are conditioned for their position and shape.

A hermetic slide device of a cup shape or fluid-tight hat is incorporated in the suction button assembly of JP-A 10-028670. When the button device is depressed halfway, an upper plate having a suction hole of the slide device is closed with an O-ring or packing contained in the button device. As a position of a piston is changed over in connection with the button device, the suction channel is set in series with the instrument channel for carry out suction through the instrument channel.

In the suction button assembly, the halfway depression of the button device or top cap device is utilized for channel selection and changeover of leaking by closing the vent hole. A shift of the button device is transmitted to the piston unit by means of the O-ring or seal packing so as to shift the piston unit. There occurs a problem in a difference in force of push between the halfway position and the down position because the piston unit is shifted with deformation of the packing. A difference in the shift of the packing occurs between the two states.

It is impossible correctly to stop the piston unit in each of the halfway position and the down position due to the difference in the shift of the piston unit with the button device. There is a requirement of a section of play in the stroke as a tolerable component in consideration of incorrectness in positioning the piston unit. Depression stroke of the piston unit with the button device must be sufficiently large according to the play. A doctor or operator may feel poor operability of the button device when he or she manually touches the button device with which the depression stroke of the piston unit is very large unlike a well-known switching valve assembly.

### SUMMARY OF THE INVENTION

In view of the foregoing problems, an object of the present invention is to provide a switching valve assembly for endoscope, in which depression stroke of a piston unit can be reduced, and in which flow lines can be changed over reliably by depressing operation of the piston unit. The invention is defined in the independent claim 1. In an exemplary embodiment, a switching valve assembly for an endoscope is provided, including a cylinder, a piston unit disposed in the cylinder in a slidable manner, a button device secured to an upper end of the piston unit, a flow channel formed in the piston unit, plural flow openings formed in the cylinder, wherein the piston unit is set in an initial position at an upper end of the cylinder, a down position, and a halfway position predetermined between the initial position and the down position upon operation of the button device, and changes over plural flow lines between communication and interruption by changing a combination of the flow openings with the flow channel for communication. The switching valve assembly includes a cylinder cap device, secured to the cylinder under the button device, for partially covering the piston unit. A slide device of a cup shape is contained in the cylinder cap device, has a receiving hole, receives entry of the piston unit, guides the piston unit in a longitudinal direction thereof, and slides in a downward direction in the cylinder cap device when the button device is pushed in the downward direction. Air-tight packing is fitted on the slide device, for contacting an inner surface of the cylinder cap device in an air-tight manner. An end ring keeps the slide device contained in the cylinder cap device. A first coil spring is disposed between the slide device and the cylinder cap device, for biasing the slide device in an upward direction from the cylinder. A second coil spring is disposed between the button device and the slide device, for biasing the button device in the upward direction with smaller force of bias than the first coil spring. A first contact surface is disposed on the button device, and opposed to the slide device. A second contact surface is disposed on the slide device, for contacting the first contact surface when the piston unit is slid to the halfway position by shift of the button device. Seal packing is associated with one of the first and second contact surfaces, for deforming resiliently to seal the first and second contact surfaces together in an air-tight manner upon contacting one another. A vent hole is formed in the slide device through the second contact surface, surrounded by the seal packing, for connection with one of the plural flow lines to come open to atmosphere.

Each of the first and second contact surfaces is constituted by a wall of metal. Consequently, depression stroke of a piston unit can be reduced, because of the use of the seal packing and the first and second contact surfaces sealed together for a correctly slidable form of the piston unit. The flow lines can be changed over reliably by depressing operation of the piston unit. Furthermore, a regulating device prevents the piston unit from rotating relative to the slide device.

The regulating device includes a first regulating flat surface formed by chamfering a peripheral surface of an end rod which is disposed in the piston unit at an upper end thereof. A second regulating flat surface is formed inside the receiving hole, for tightly contacting the first regulating flat surface for engagement.

The down position of the piston unit is adapted to expansion or compression of a balloon of the endoscope with water, and the halfway position of the piston unit is adapted to supply or suction of fluid through a nozzle of the endoscope in a body cavity.

The down position is adapted to compression of the balloon, and the halfway position is adapted to suction of the fluid in the body cavity.

### BRIEF DESCRIPTION OF THE DRAWINGS

The above objects and advantages of the present invention will become more apparent from the following detailed description when read in connection with the accompanying drawings, in which:
Fig. 1 is an explanatory view in a section, illustrating an endoscope;
Fig. 2 is a vertical section illustrating a suction button assembly;
Fig. 3 is a front elevation illustrating the suction button assembly;
Fig. 4 is a top plan illustrating a cylinder;
Fig. 5 is a perspective view illustrating a cylinder cap device;
Fig. 6 is a bottom perspective view illustrating the cylinder cap device;
Fig. 7 is a perspective view illustrating a slide device of a cup shape;
Fig. 8 is a bottom perspective view illustrating the slide device;
Fig. 9 is a cross section illustrating the suction button assembly taken on line IX-IX in Fig. 2;
Fig. 10 is a cross section illustrating the suction button assembly taken on line X-X in Fig. 2;
Fig. 11 is a cross section illustrating the suction button assembly taken on line XI-XI in Fig. 2;
Fig. 12 is a chart illustrating relationships between the cylinder and the piston unit for rotational regulation;
Fig. 13 is a vertical section illustrating the suction button assembly in an initial state of the button device;
Fig. 14 is a vertical section illustrating the suction button assembly in a state of halfway depressing the button device;
Fig. 15 is a vertical section illustrating the suction button assembly in a state of fully depressing the button device;
Fig. 16 is a vertical section illustrating another preferred suction button assembly in which the slide device does not have annular seal packing.

### DETAILED DESCRIPTION OF THE PREFERRED EMBODIMENT(S) OF THE PRESENT INVENTION

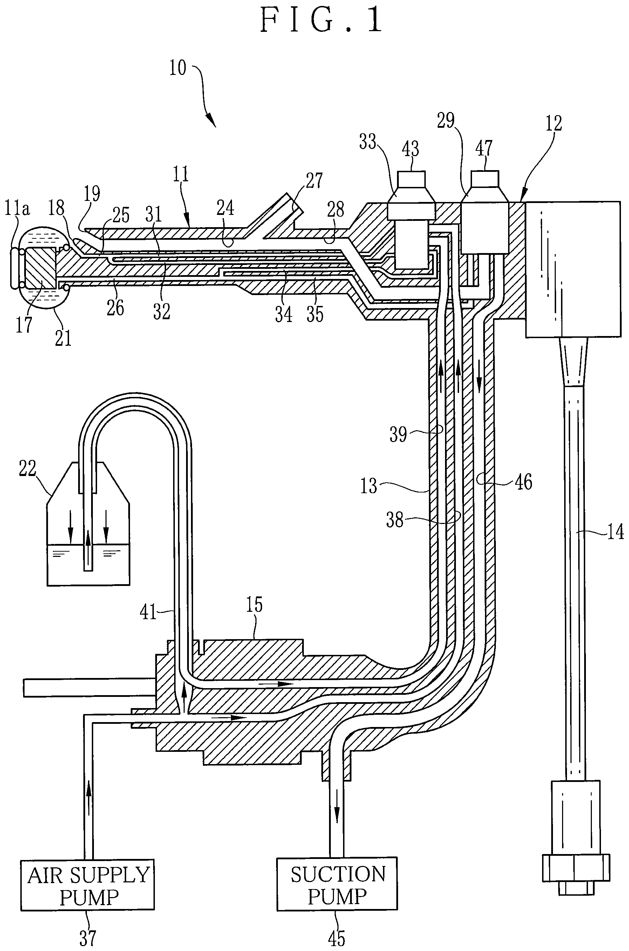

In Fig. 1, an ultrasonic endoscope 10 includes a section of an elongated tube 11, a handle device 12, a universal cable 13 or connection tube, and a connection cable 14. The elongated tube 11 is entered in a body cavity of a patient. The handle device 12 is a basic portion at which the universal cable 13 and the connection cable 14 are connected to the handle device 12. A connector 15 is disposed at a proximal end of the universal cable 13 for connection with a processing apparatus for the endoscope with a light source apparatus. A proximal end of the connection cable 14 is connected to the processing apparatus (not shown).

The elongated tube 11 has a circular shape as viewed in a cross section, and is flexible. There is a head assembly 11a of the elongated tube 11. The head assembly 11a includes an ultrasonic transducer array 17, a CCD image sensor (not shown), an air/water supply nozzle 18, and a suction nozzle 19 or instrument opening. The ultrasonic transducer array 17 operates to form an ultrasonic image. The CCD image sensor forms an endoscopic image. The air/water supply nozzle 18 washes an objective system for imaging (not shown). The suction nozzle 19 is a distal opening for protrusion of a medical instrument such as a forceps, and also is a suction opening for drawing fluid such as blood or body fluid.

A resilient balloon 21 is secured to the head assembly 11a in a removable manner. The balloon 21 is initially compressed to contact the outer surface of the head assembly 11a tightly before entry into the body cavity. A water supply source 22 or water supply tank supplies water to the balloon 21 for expansion for the purpose of applying ultrasonic waves from the ultrasonic transducer array 17. The balloon 21 operates for tightening the contact of the head assembly 11a on a wall of the body cavity, and prevents ultrasonic waves and echo from attenuating with air. After the expansion, the balloon 21 is compressed again by discharge of water. An example of material of the balloon 21 is latex rubber and the like.

An instrument channel 24, a fluid supply channel 25 and a balloon channel 26 are formed through the elongated tube 11 and the handle device 12. A distal end of the instrument channel 24 is the suction nozzle 19. A distal end of the fluid supply channel 25 is the air/water supply nozzle 18. A distal end of the balloon channel 26 extends to the inner space of the balloon 21. Note that in Fig. 1, portions other than the channels are hatched for the purpose of clarifying the channels.

A proximal instrument opening 27 is formed in the elongated tube 11, and is an open end of the instrument channel 24. A seal cap (not shown) is fitted on the proximal instrument opening 27 for closing except for entry of a medical instrument or forceps. A suction channel 28 is a branch conduit of the instrument channel 24. A suction button assembly 29 or switching valve assembly is associated with the handle device 12. The suction channel 28 is connected to the suction button assembly 29.

An air channel 31 and a water channel 32 are branches extending from a proximal end of the fluid supply channel 25. On the handle device 12 is an air/water supply button assembly 33 or switching valve assembly, to which the air channel 31 and the water channel 32 are connected. A balloon expansion channel 34 and a balloon compression channel 35 are branches extending from a proximal end of the balloon channel 26. The balloon expansion channel 34 is connected to the air/water supply button assembly 33. The balloon compression channel 35 is connected to the suction button assembly 29.

There is an air supply conduit 38, which extends to an air supply source 37 or air supply pump. A water supply conduit 39 extends to the water supply source 22. Various flow lines are connected to the air/water supply button assembly 33, including the air channel 31, the water channel 32, the balloon expansion channel 34, the air supply conduit 38 and the water supply conduit 39. The air supply source 37 operates constantly during the ultrasonic imaging.

A branch conduit 41 is a branch from a proximal end of the air supply conduit 38 and extends through the connector 15. The branch conduit 41 is connected to a port of the water supply source 22. A proximal end of the water supply conduit 39 is disposed within the water supply source 22 by way of the branch conduit 41. Inner pressure of the water supply source 22 is raised by air supply through the branch conduit 41 from the air supply source 37. Water from the water supply source 22 is drawn to the water supply conduit 39.

The air/water supply button assembly 33 is a two-step button. A button device 43 or top cap device is an upper element of the air/water supply button assembly 33. A vent channel (not shown) is formed through the button device 43. When the button device 43 is not depressed, the air/water supply button assembly 33 shuts off the water supply conduit 39, and sets the air supply conduit 38 in series with the vent channel of the button device 43. Air from the air supply conduit 38 is leaked through the vent channel of the air/water supply button assembly 33. A finger of an operator closes the vent channel, to set the air supply conduit 38 in series with the air channel 31 in a state of shut-off of the water supply conduit 39, in a manner described in JP-A 10-028670 (corresponding to JP-B 3017957). Thus, the air is caused to flow to the air channel 31 and ejected through the air/water supply nozzle 18.

When the button device 43 is depressed halfway, the air/water supply button assembly 33 shuts off the air supply conduit 38, and sets the water supply conduit 39 in series with only the water channel 32. Water from the water supply conduit 39 is passed through the water channel 32 and ejected from the air/water supply nozzle 18. When the button device 43 is depressed fully, the air/water supply button assembly 33 sets the water supply conduit 39 in series with only the balloon expansion channel 34 while the air supply conduit 38 is kept shut off. Therefore, water from the water supply conduit 39 is passed through the balloon expansion channel 34 and supplied into the balloon 21.

There is a discharge conduit 46 having proximal and distal ends. The proximal end is connected to a suction pump 45. The distal end is connected to the suction button assembly 29 as well as the suction channel 28 and the balloon compression channel 35. The suction pump 45 operates for suction during the ultrasonic imaging. The suction button assembly 29 is a two-step button in a manner similar to the air/water supply button assembly 33.

When a button device 47 or top cap device is not depressed, the suction button assembly 29 sets the discharge conduit 46 open to the atmosphere. If the discharge conduit 46 is not open, load to the suction pump 45 may increase, as the suction pump 45 operates constantly. As the discharge conduit 46 is set open to the atmosphere, overload to the suction pump 45 can be prevented.

When the button device 47 is depressed halfway, the suction button assembly 29 sets the discharge conduit 46 in series only with the suction channel 28. Force of the suction with negative pressure rises in the suction channel 28 and the instrument channel 24 to draw fluid through the suction nozzle 19. When the button device 47 is depressed fully, the suction button assembly 29 sets the discharge conduit 46 in series only with the balloon compression channel 35. Thus, water is discharged from the balloon 21 by increasing the force of the suction with negative pressure in the balloon compression channel 35 and the balloon channel 26.

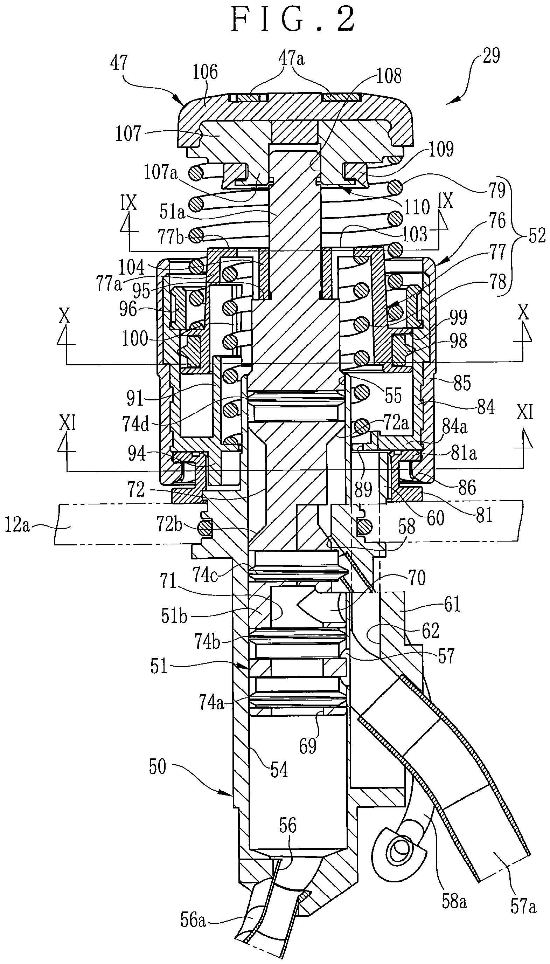

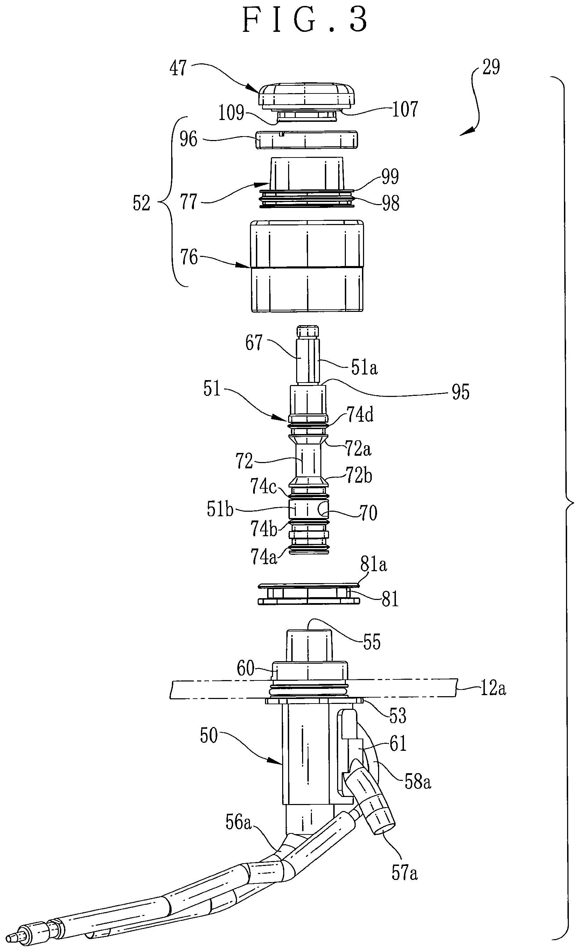

In Figs. 2 and 3, the suction button assembly 29 includes a cylinder 50, a piston unit 51, and a housing unit 52 of a cup shape or cylinder cap unit. The cylinder 50 is firmly secured to the handle device 12. The piston unit 51 is contained in the cylinder 50 in a slidable manner. The housing unit 52 is retained on the cylinder 50, and positions the piston unit 51 in a halfway position and a down position lower than the halfway position.

The cylinder 50 is formed from metal. The suction channel 28, the balloon compression channel 35, and the discharge conduit 46 are connected to the cylinder 50. A cylinder passage 54 of a multi-nozzle type is formed through the cylinder 50 and extends longitudinally. An end opening 55 is an open end of the cylinder passage 54. A flow port 56 or suction port is another open end of the cylinder passage 54, and communicates with the suction channel 28. A suction channel coupling 56a or nozzle is disposed to couple the flow port 56 to the suction channel 28.

A flow opening 57 or nozzle opening and a drain port 58 are formed in an inner wall of the cylinder passage 54. The flow opening 57 communicates with the discharge conduit 46 in a downward direction. The drain port 58 communicates with the balloon compression channel 35. A discharge channel coupling 57a or nozzle of the flow opening 57 is connected to the discharge conduit 46. A drain channel coupling 58a or nozzle of the drain port 58 is connected to the balloon compression channel 35.

A receiving threaded portion 60 is an end portion of the cylinder 50, and contacts a lower portion of the housing unit 52. See Fig. 3. A discharge chamber 61 is disposed for connection of the flow opening 57 on the outside of the cylinder 50, and used for connection with the discharge channel coupling 57a. The discharge chamber 61 extends longitudinally toward an end of the cylinder 50, and is connected with the receiving threaded portion 60. In Fig. 2, the discharge chamber 61 is partially cut away for clarifying the connected portions of the cylinder 50 and the balloon compression channel 35.

A fluid channel 62 is formed through the discharge chamber 61. The flow opening 57 and the discharge channel coupling 57a are open at a lower end of the fluid channel 62. An upper end of the fluid channel 62 is connected to the receiving threaded portion 60 as indicated by the phantom line of Fig. 2.

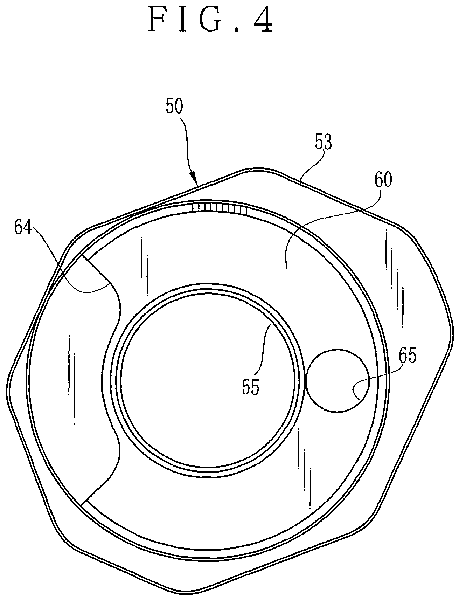

In Fig. 4, a regulating recess 64 is formed with the receiving threaded portion 60 by chamfering an outer wall, and operates for rotational regulation with the housing unit 52. A cylinder vent hole 65 is formed in an upper wall of the receiving threaded portion 60, and communicates with the fluid channel 62. A male thread (not shown) is formed around the receiving threaded portion 60. A threaded ring 81 as a retaining ring is helically engaged with the male thread. See Fig. 3. A support flange 53 is formed with a lower portion of the receiving threaded portion 60. A handle housing 12a of the handle device 12 has a lower surface. The support flange 53 contacts the lower surface. The cylinder 50 is fixedly secured to the handle housing 12a by helical engagement of the threaded ring 81 with the male thread.

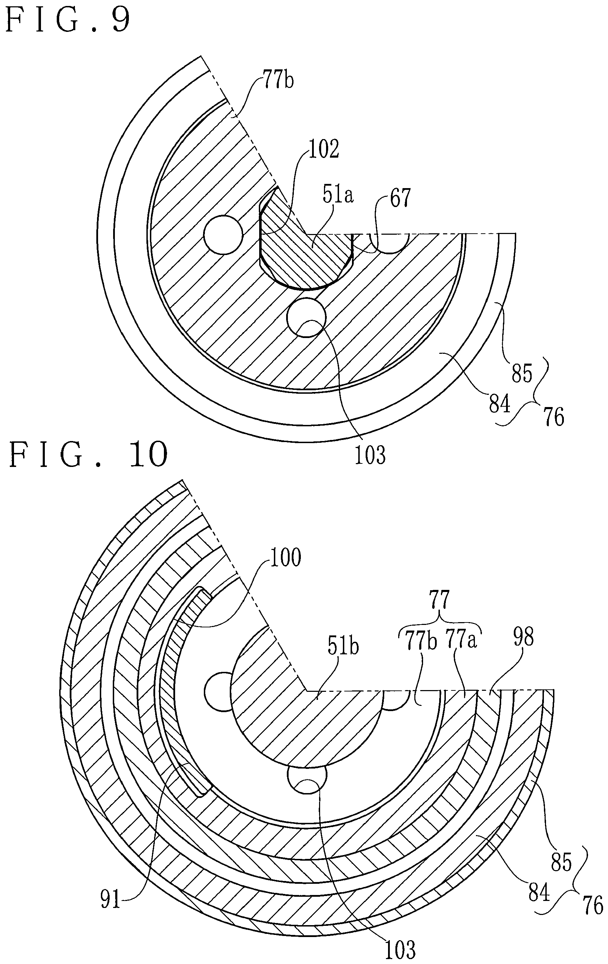

In Figs. 2 and 3, the piston unit 51 is formed from metal, and includes an end rod 51a, and a valve head 51b or piston rod or valve sleeve. The end rod 51a protrudes from the end opening 55. The valve head 51b is always contained in the cylinder passage 54. A diameter of the end rod 51a is smaller than that of the valve head 51b. A regulating flat surface 67 is formed by chamfering the end rod 51a, and operates for rotational regulation of the housing unit 52. See Figs. 3 and 9.

The button device 47 is fixed on an upper end of the end rod 51a, has a disk shape, and is depressed for halfway depression and full depression. A target indicia 47a is formed on an upper surface of the button device 47 for expressing a depression position of a finger.

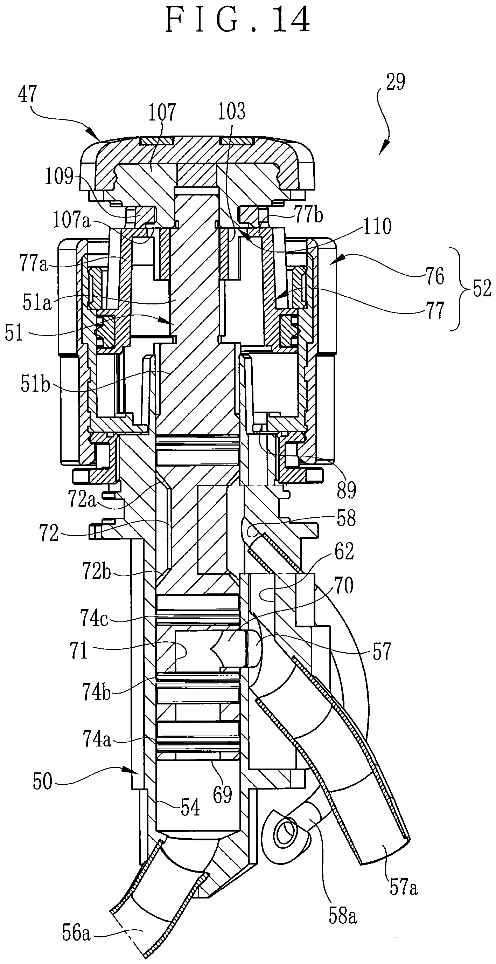

The valve head 51b includes a lower opening 69, a valve opening 70 or side opening, and a flow channel 71 for communication between the lower opening 69 and the valve opening 70. The valve opening 70 is so disposed as to be aligned with the flow opening 57 upon halfway depression. See Fig. 14.

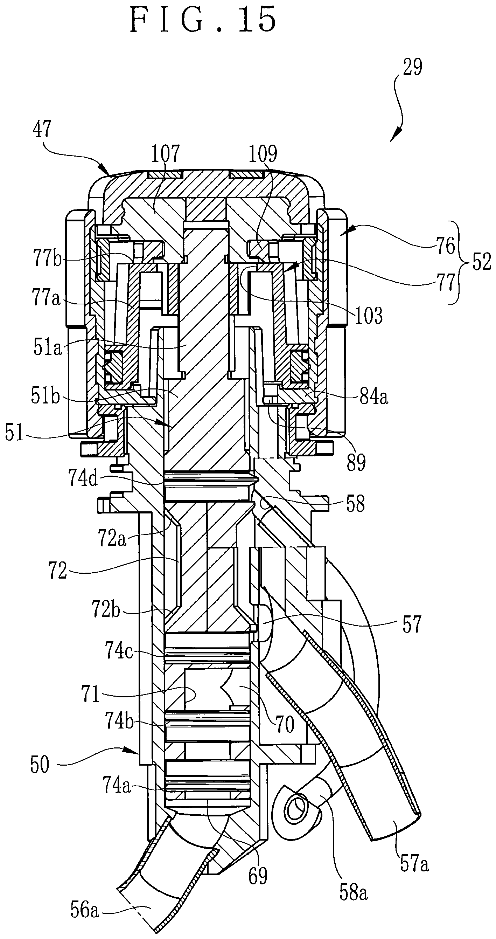

An annular channel 72 or annular flow opening is formed in a wall of the valve head 51b, disposed away from the valve opening 70 toward the end rod 51a, and extends in the longitudinal direction of the piston unit 51. The annular channel 72 includes an upper channel end 72a and a lower channel end 72b. The annular channel 72 has such a length that, when the piston unit 51 is depressed fully, the upper channel end 72a is opposed to the drain port 58, and that the lower channel end 72b is opposed to the flow opening 57. See Fig. 15. In Fig. 2, the annular channel 72 is partially cut away in the drawing for the purpose of clarifying the drain port 58.

A first packing 74a, second packing 74b, third packing 74c and fourth packing 74d are fitted on a wall of the valve head 51b. The first packing 74a is disposed at a lower end of the valve head 51b. The second packing 74b and the third packing 74c are disposed on the valve head 51b so that the valve opening 70 is positioned between those. The fourth packing 74d is disposed on the valve head 51b so that the annular channel 72 is positioned between the third packing 74c and the fourth packing 74d.

The piston unit 51 slides between an initial position and a down position. The button device 47, when the piston unit 51 is in the initial position, is not depressed but disposed the farthest from the end opening 55, and when the piston unit 51 is in the down position (full depression), is depressed fully, disposed the nearest to the end opening 55, and prevented from further moving down.

The piston unit 51, when in the initial position, is in a shut-off state to disconnect the flow opening 57 from the flow port 56 and the drain port 58 by use of the wall of the valve head 51b and the packing 74a-74d. The piston unit 51, when in the down position, is in a balloon deflation state of Fig. 15 for communicating the drain port 58 with the flow opening 57 by use of the annular channel 72. The piston unit 51, when slid to a halfway position between the initial position and down position by operating the button device 47, is in a suction state of Fig. 14 for communicating only the flow port 56 with the flow opening 57 by use of the flow channel 71.

The housing unit 52 includes a cylinder cap device 76 of a cup shape or housing wall, an air-tight slide device 77 of a cup shape or guide cup by way of an intermediate casing, a first compression coil spring 78, and a second compression coil spring 79. The cylinder cap device 76 is secured to one end of the cylinder 50 and disposed around the end opening 55. The air-tight slide device 77 is disposed inside the cylinder cap device 76. Portions of the housing unit 52 have a larger diameter than the end of the cylinder 50.

The cylinder cap device 76 is secured to an end of the cylinder 50 by the threaded ring 81. A receiving hole is formed in the threaded ring 81 for entry of the receiving threaded portion 60. A female thread (not shown) is provided on the inside of the receiving hole. The receiving threaded portion 60 or male thread is helically engaged with the female thread of the threaded ring 81 to connect the threaded ring 81 to the end of the cylinder 50. An annular flange 81a is formed on one end of the threaded ring 81.

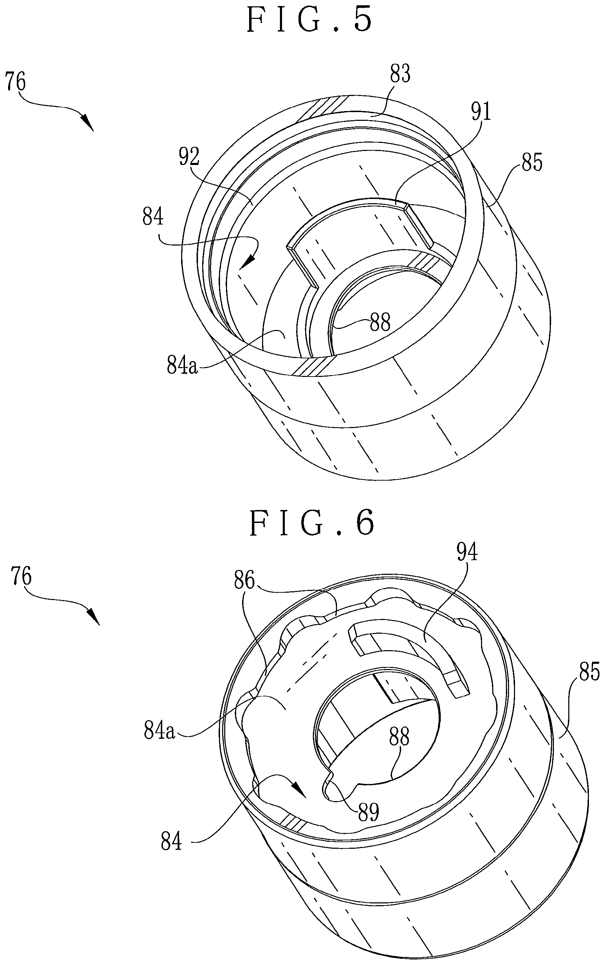

The cylinder cap device 76 includes a cup sleeve 84 and a cover sleeve 85. The cup sleeve 84 is formed from metal. The cover sleeve 85 is formed from resin, and covers the cup sleeve 84. A cup opening 83 is an upper open end of the cup sleeve 84 as illustrated in Fig. 5. A lower end of the cover sleeve 85 extends toward the cylinder 50 down under a bottom of the cup sleeve 84. Plural engaging teeth 86 are formed with an inner surface of the end of the cover sleeve 85 for engagement with the annular flange 81a. See Fig. 6. Thus, the cup sleeve 84 is connected to the cylinder 50 by the cover sleeve 85 and the threaded ring 81.

A bottom plate 84a of the cup sleeve 84 contacts an upper surface of the receiving threaded portion 60. A receiving hole 88 of Figs. 5 and 6 is formed through the bottom plate 84a for receiving entry of the cylinder 50. A cup vent hole 89 of Fig. 6 is formed in the bottom plate 84a, and opposed to the cylinder vent hole 65. Thus, air can flow between the inside of the cup sleeve 84 and the fluid channel 62 of the cylinder 50.

A sleeve wall 77a is included in the air-tight slide device 77 as illustrated in Figs. 2 and 3. In Fig. 5, a regulating ridge 91 is formed on a surface of the bottom plate 84a opposed to the air-tight slide device 77, and projects toward the sleeve wall 77a. An annular recess 92 for support is formed in an inner surface of the cup sleeve 84 and disposed under the cup opening 83.

In Fig. 6, a regulating ridge 94 is formed on a surface of the bottom plate 84a opposed to the cylinder 50, and projects toward the receiving threaded portion 60. The regulating ridge 94 is engaged with the regulating recess 64 of the receiving threaded portion 60.

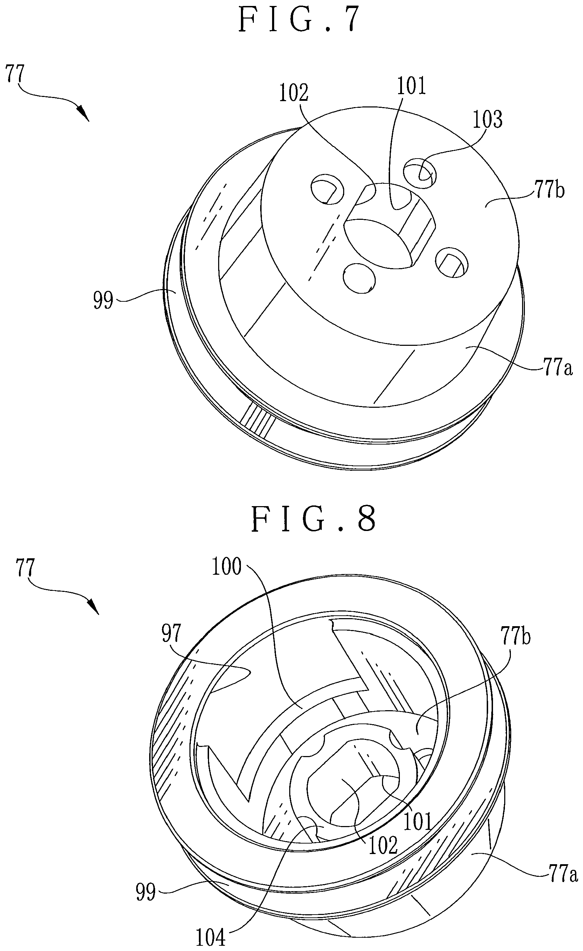

In Figs. 2 and 3, the air-tight slide device 77 is kept slidable inside the cup sleeve 84. An end ring 96 is secured to the annular recess 92, and maintains the air-tight slide device 77 inside the cup sleeve 84 without drop. The air-tight slide device 77 includes a cup plate portion 77b and the sleeve wall 77a. The sleeve wall 77a is cylindrical and extends longitudinally along the piston unit 51. The cup plate portion 77b is formed on an upper end of the sleeve wall 77a. A cup opening 97 is a lower open end of the sleeve wall 77a. See Fig. 8. Diameters of the sleeve wall 77a and the cup opening 97 are predetermined larger than an inner diameter of the end opening 55 and smaller than an inner diameter of the cup sleeve 84.

An annular seal packing 98 (air-tight packing) is fitted on the lower end of the sleeve wall 77a. A packing support 99 with two ring flanges of the sleeve wall 77a supports the annular seal packing 98. The annular seal packing 98 contacts an inner surface of the cup sleeve 84 and prevents leakage of air through a gap between an outer surface of the sleeve wall 77a and the inner surface of the cup sleeve 84. The ring flanges of the packing support 99 have an outer diameter larger than an inner diameter of the end ring 96.

A regulating recess 100 is formed in a second end portion of the sleeve wall 77a for engagement with the regulating ridge 91. See Fig. 8. A length of the regulating recess 100 is sufficiently larger than the regulating ridge 91 in the longitudinal direction of the piston unit 51 in order to keep the air-tight slide device 77 slidable without fail.

In Fig. 7, a receiving hole 101 is formed in the cup plate portion 77b and receives entry of the end rod 51a. A regulating flat surface 102 is formed flatly in the receiving hole 101 and is engaged with the regulating flat surface 67 of the end rod 51a. In short, the inner surface of the receiving hole 101 is non-circular in the presence of the regulating flat surface 102.

Plural cup vent holes 103 are formed in the cup plate portion 77b and disposed around the receiving hole 101. The cup vent holes 103 are open to the atmosphere. Thus, the discharge conduit 46 is caused to communicate with the atmosphere by the discharge channel coupling 57a, the fluid channel 62, the cup vent hole 89, the cup sleeve 84, the air-tight slide device 77, and the cup vent holes 103.

In Fig. 8, a guide sleeve 104 is formed on the cup plate portion 77b, and extends down toward the end opening 55. An upper end of the guide sleeve 104 has the receiving hole 101. The end rod 51a is inserted in the guide sleeve 104 in a slidable manner. As the guide sleeve 104 has a predetermined length in the longitudinal direction, the air-tight slide device 77 can be supported on the end rod 51a without an inclination. Offsetting of the piston unit 51 can be prevented when the piston unit 51 slides.

An inner diameter of the guide sleeve 104 is smaller than a diameter of the valve head 51b. A receiving surface 95 for retention is formed with a stepped shape on the valve head 51b. See Fig. 2. A lower end of the guide sleeve 104 contacts the receiving surface 95. This operates to keep the piston unit 51 positioned in the cylinder 50 without dropping by use of the air-tight slide device 77, the end ring 96, the cylinder cap device 76 and the threaded ring 81.

In Fig. 2, the air-tight slide device 77 is slidable between an upper position (protrusion position) and a lower position (storage position). When the air-tight slide device 77 is in the upper position, an end of the packing support 99 contacts the end ring 96 to protrude the cup plate portion 77b from the cup opening 83. When the air-tight slide device 77 is in the lower position of Fig. 15, a second end of the sleeve wall 77a contacts the bottom plate 84a to contain the cup plate portion 77b in the cup opening 83. The air-tight slide device 77 is in the upper position while the piston unit 51 slides from the initial position to the halfway position.

When the piston unit 51 slides from the halfway position toward the down position upon depressing the button device 47, the air-tight slide device 77 is slid by the button device 47 from the upper position to the lower position. When the piston unit 51 comes to the down position, the air-tight slide device 77 is slid to the lower position, and prevents the button device 47 and the piston unit 51 from being pushed further.

The first compression coil spring 78 is disposed between the bottom plate 84a and the cup plate portion 77b in a compressed state along the piston unit 51 with a shorter length than its free state. The piston unit 51 is inserted through the first compression coil spring 78. The first compression coil spring 78 biases the cup plate portion 77b to protrude from the cup opening 83 so as to keep the air-tight slide device 77 in the upper position.

The second compression coil spring 79 is disposed between the packing support 99 and the button device 47 in a state compressed in the direction along the piston unit 51 with a reduced length. The piston unit 51 is inserted in the second compression coil spring 79. The second compression coil spring 79 biases the button device 47 in a direction to protrude from the receiving hole 101. Force of bias of the second compression coil spring 79 is structurally smaller than that of the first compression coil spring 78. When the button device 47 is depressed, at first the second compression coil spring 79 starts being deformed, then the first compression coil spring 78 starts being deformed. Consequently, the button device 47 can be moved and stopped in the halfway position owing to the difference in the force of bias between the compression coil springs 78 and 79.

The compression coil springs 78 and 79 are arranged inside the housing unit 52 so that the air-tight slide device 77 is disposed between those. The first compression coil spring 78 inside the air-tight slide device 77 and the second compression coil spring 79 outside the air-tight slide device 77 constitute such a double structure that the housing unit 52 can have a small height because the compression coil springs 78 and 79 can be accommodated compactly.

The piston unit 51 is maintained in the initial position by the bias of the compression coil springs 78 and 79. To slide the piston unit 51 from the initial position to the halfway position, the button device 47 must be pushed against the second compression coil spring 79. To slide the piston unit 51 from the halfway position to the down position (full depression), the button device 47 must be pushed against the compression coil springs 78 and 79. In short, the force of the bias applied to the button device 47 changes during the slide of the piston unit 51 from the initial position to the down position.

The button device 47 includes a cap top portion 106 of a disk shape and a button head or pressure ring 107 of metal. The cap top portion 106 is formed from resin. The button head 107 is secured to a lower surface of the cap top portion 106, and tightly contacts the cup plate portion 77b when the piston unit 51 is located between the halfway position and the down position. A ring projection 107a projects from the button head 107 toward the cup plate portion 77b. A screw hole 108 with a female thread (not shown) as retention mechanism is formed in the ring projection 107a. A male thread (not shown) of the end rod 51a is helically engaged with the screw hole 108, to fasten the button head 107 to the end rod 51a.

A pressure surface 110 or closure surface is formed with one end of the ring projection 107a, has an annular shape, and contacts the cup plate portion 77b. The pressure surface 110 covers and closes the cup vent holes 103 upon contacting the cup plate portion 77b. When the piston unit 51 is depressed fully, the pressure surface 110 contacts the cup plate portion 77b and the sleeve wall 77a contacts the bottom plate 84a. Each of those elements is formed from metal, so that errors in the piston longitudinal direction can be reduced in relation to the valve opening 70 or the annular channel 72 of the piston unit 51 in the down position.

An annular groove is formed in the ring projection 107a. A seal packing 109 of a ring shape is fitted in the annular groove, and formed from resilient material. An end of the seal packing 109 extends in a direction toward the cup plate portion 77b further than the pressure surface 110, and has a gradually decreasing thickness. When the pressure surface 110 contacts the cup plate portion 77b, the end of the seal packing 109 tightly contacts the cup plate portion 77b in a resiliently deformed state. Even when the cup vent holes 103 are not completely closed with the pressure surface 110, it is possible to disconnect the cup vent holes 103 from the atmosphere for shut-off of air. As a result, the discharge conduit 46 is shut off from the atmosphere.

Fig. 9 is a section taken on line IX-IX in Fig. 2. The regulating flat surface 67 of the end rod 51a is engaged with the regulating flat surface 102 of the receiving hole 101 of the cup plate portion 77b, so that the air-tight slide device 77 is rotationally regulated on the piston unit 51 about its axis.

Fig. 10 is a section taken on line X-X in Fig. 2. The regulating ridge 91 of the cup sleeve 84 is engaged with the regulating recess 100 of the sleeve wall 77a, so that the air-tight slide device 77 is rotationally regulated on the cylinder cap device 76 about its axis.

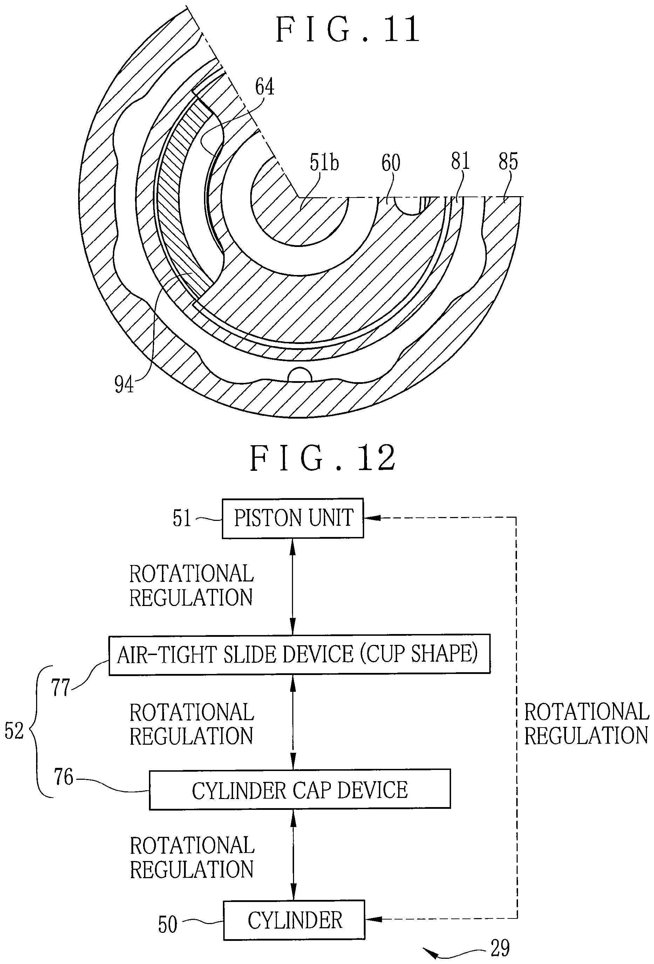

Fig. 11 is a section taken on line XI-XI in Fig. 2. The regulating ridge 94 of the bottom plate 84a is engaged with the regulating recess 64 of the receiving threaded portion 60, so that the cylinder cap device 76 is rotationally regulated on the cylinder 50 about its axis.

In Fig. 12, the rotational regulation is carried out between the piston unit 51 and the air-tight slide device 77, between the air-tight slide device 77 and the cylinder cap device 76, and between the cylinder cap device 76 and the cylinder 50. Thus, the piston unit 51 is rotationally regulated in the cylinder 50 by the housing unit 52 indirectly. It is possible reliably to align the valve opening 70 with the flow opening 57 when the piston unit 51 is slid to the halfway position for the suction state, owing to the positions and shapes of the regulating flat surface 67, the regulating ridges 91 and 94, the regulating recesses 64 and 100 and the regulating flat surface 102.

The operation of the ultrasonic endoscope 10 is described now specifically in relation to the suction button assembly 29. In the endoscopic examination, a CCD image sensor and the ultrasonic transducer array 17 operate constantly. The air supply source 37 supplies air. The suction pump 45 carries out suction. When those devices are ready, the elongated tube 11 is entered in a body cavity of a patient, for example gastrointestinal tract, to start imaging. The balloon 21 is completely empty by removal of water, and remains compressed to contact the head assembly 11a tightly.

At first, a body part in the gastrointestinal tract is imaged endoscopically with the image sensor. The button device 43 of the air/water supply button assembly 33, if required for a type of the body part or for washing an imaging window (not shown) of the head assembly 11a, is operated to supply air and water through the air/water supply nozzle 18. When a doctor or operator wishes to change over the imaging, the endoscopic imaging is changed over to ultrasonic imaging, typically for more precise imaging upon discovery of a lesion in the gastrointestinal tract or the like.

For imaging with the ultrasonic endoscope, the button device 43 is depressed fully to supply water from the water supply source 22 through the water supply conduit 39, the balloon expansion channel 34 and the balloon channel 26 into the balloon 21, which is expanded. Various known methods for adjusting a flow rate of the water to the balloon 21 can be used in the embodiment. After the expansion, the balloon 21 is set in tight contact with an object of interest, for example, a lesion in a body part. An ultrasonic image of the object of interest is formed.

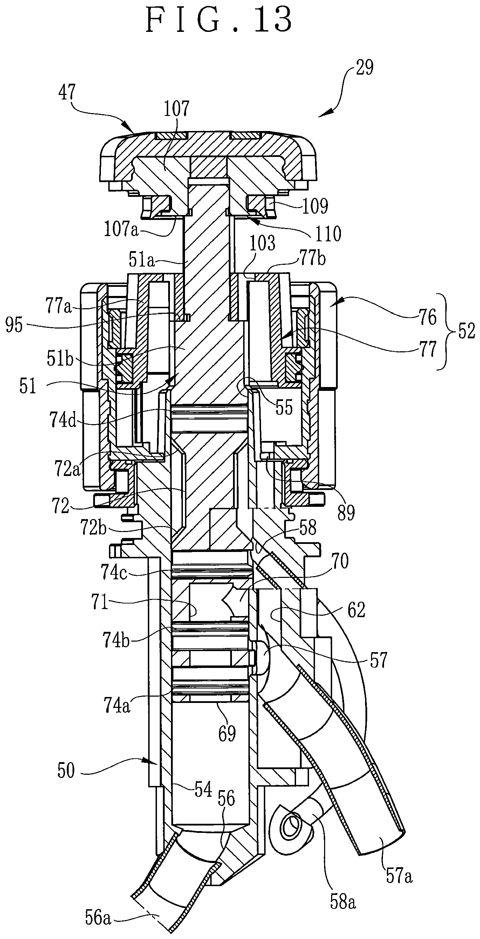

In Fig. 13, the button device 47 of the suction button assembly 29 is not pushed in a normal state without suction or drain of a balloon in the ultrasonic imaging or endoscopic imaging. The piston unit 51 is kept in the initial position for shut-off by the compression coil springs 78 and 79. As the flow channel 71 and the annular channel 72 have not been positioned for setting the flow opening 57 for communication of the flow port 56 with the drain port 58, the discharge conduit 46 is disconnected from the suction channel 28 and the balloon compression channel 35. There is no suction through the suction nozzle 19 or no drain of the balloon 21.

When the piston unit 51 is in the initial position, the cup vent holes 103 in the cup plate portion 77b are open. The flow opening 57 becomes open to the atmosphere through the fluid channel 62, the cup vent holes 89 and 103 and the like. It is possible to prevent overload to the suction pump 45 even when no suction is carried out through the suction nozzle 19 or when no removal of water is carried out from the balloon 21.

If suction of blood or body fluid is required in the imaging, the button device 47 is depressed halfway to slide in the piston unit 51 through the end opening 55. Before the piston unit 51 reaches the halfway position, force of bias of the second compression coil spring 79 is applied to the button device 47. After the piston unit 51 slides down past the halfway position, force of bias of both the compression coil springs 78 and 79 is applied to the button device 47. In short, the force of bias applied to the button device 47 increases, so that the piston unit 51 can be stopped suitably in the halfway position.

In Fig. 14, the piston unit 51 is changed over from the inactive state to the suction state upon stop in the halfway position. In the suction state, the valve opening 70 of the flow channel 71 becomes aligned with the flow opening 57. The lower channel end 72b of the annular channel 72 has not reached the position of the flow opening 57. Also, the third packing 74c comes to a location between the flow opening 57 and the drain port 58. As a result, only the flow port 56 comes to communicate with the flow opening 57.

When the flow port 56 comes to communicate with the flow opening 57, the discharge conduit 46 is set in series with the suction channel 28 and the instrument channel 24 by the flow channel 71 and other channels. When the piston unit 51 is in the halfway position, the pressure surface 110 of the ring projection 107a and the seal packing 109 contact the cup plate portion 77b tightly to close the cup vent holes 103. The discharge conduit 46 is shut off from the atmosphere, to raise suction force of the negative pressure in the channels including the discharge conduit 46 and the suction nozzle 19. Thus, fluid is drawn through the suction nozzle 19 by the suction. The fluid is passed through the instrument channel 24, the suction channel 28, the cylinder passage 54, the flow channel 71 and the discharge conduit 46 and discharged from the ultrasonic endoscope 10.

Consequently, the valve opening 70 of the piston unit 51 is always aligned with the flow opening 57 because the housing unit 52 prevents the piston unit 51 from rotating relative to the cylinder 50 upon changeover of the piston unit 51 to the suction state. A flow channel width determined between the valve opening 70 and the flow opening 57 is maximized for flow of fluid, so as to increase the performance of suction of the suction button assembly 29 very effectively. Also, it is unnecessary to dispose a rotationally regulating element to each one of a wall of the piston unit and an inner wall of the cylinder. The degree of freedom of defining positions of the flow channel 71, the annular channel 72 and other channels can be high in the piston unit 51. A size of the piston unit 51 or the suction button assembly 29 can be reduced.

To discontinue the suction, the button device 47 is left without depression. The second compression coil spring 79 returns the piston unit 51 to the initial position of Fig. 13.

When the ultrasonic imaging is terminated, the button device 47 is depressed fully to slide the piston unit 51 down into the end opening 55. Before the piston unit 51 comes to reach the halfway position, force of bias of the second compression coil spring 79 is applied to the button device 47. When the piston unit 51 moves past the halfway position, force of bias of the compression coil springs 78 and 79 is applied to the button device 47. Also, when the piston unit 51 moves down past the halfway position, pressure of the button device 47 moves the air-tight slide device 77 from the upper position to the lower position. When the button device 47 continues being pushed against the compression coil springs 78 and 79, the air-tight slide device 77 reaches the lower position, and prevents the button device 47 from moving down further. Thus, the piston unit 51 is stopped in the down position (full depression).

In Fig. 15, the piston unit 51 is in the down position for changeover to the balloon deflation state. In this state, the valve opening 70 of the flow channel 71 is offset from the flow opening 57 in the longitudinal direction of the piston unit 51. The annular channel 72 is shifted to oppose the upper channel end 72a to the drain port 58 and the lower channel end 72b to the flow opening 57. Also, the third packing 74c shifts to a point between the valve opening 70 and the flow opening 57. Only the drain port 58 is set in series with the flow opening 57.

When the drain port 58 comes to communicate with the flow opening 57, the discharge conduit 46 and the balloon compression channel 35 (and the balloon channel 26) are caused to communicate with one another by the annular channel 72. As the cup vent holes 103 are closed in a manner similar to the suction described above, the discharge conduit 46 is shut off from the atmosphere. Force of suction with the negative pressure rises within the channels including the discharge conduit 46 and the balloon channel 26. Thus, water is removed from the balloon 21 which is compressed. The removed water is drawn through the balloon channel 26, the balloon compression channel 35, the annular channel 72 and the discharge conduit 46 and drained from the ultrasonic endoscope 10.

Discharge of a predetermined amount of water from the balloon 21 is detected according to a known method of detection. In response, the button device 47 is released from being pushed. Thus, the piston unit 51 is returned to the initial position of Fig. 13 by the bias of the compression coil springs 78 and 79.

Similarly, the air/water supply button assembly 33 and the suction button assembly 29 are actuated suitably until the end of the examination with the ultrasonic endoscope 10. Supply of air and water, water supply to the balloon, suction, water drain from the balloon are carried out.

The feature of the embodiment lies in the combination of the pressure surface 110, the cup plate portion 77b (contact surfaces), the seal packing 109 and the cup vent holes 103. So it is possible to obtain a desired shift amount in the depression of the button device 47 without intermediate presence of the seal packing 109 or the like. It is possible to reduce the predetermined stroke of pushing the button device 47 for the piston unit 51, because a component of the stroke for play as a tolerable part is not required. For a user to manipulate the endoscope, manual touch of the button device 47 is considerably light because of the reduced stroke. Also, after the contact of the contact surfaces, the contact surfaces can be kept air-tight by the seal packing 109. It is possible to draw fluid or water through the suction channel or balloon compression channel reliably upon start of suction with a suction pump.

In the above embodiment, the seal packing 109 is associated with the button device 47. However, any element for sealing between the contact surfaces of metal in contact with one another can be used according to the invention. For example, the seal packing 109 can be fitted on the air-tight slide device 77 for contact with the button device 47.

Also, the air-tight slide device 77, the cup sleeve 84 and the button head 107, although formed from metal according to the above embodiment, can be formed from plastic material. It is possible correctly to stop the piston unit in each of the halfway position and down position, because of the unchanged shift amount in the manner described above.

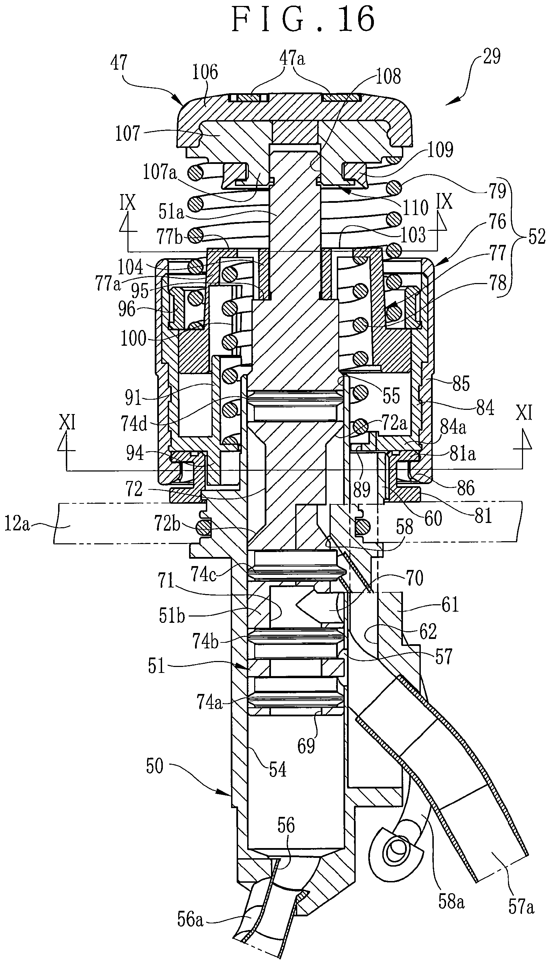

Fig. 16 illustrates another preferred example of the suction button assembly 29 in which the air-tight slide device 77 does not have the annular seal packing 98 (air-tight packing) . The air-tight slide device 77 is contained in the cylinder cap device 76 in an air-tight manner itself in a slidable form.

In the above embodiment, the button assembly is the suction button assembly 29. However, a valve assembly of the invention can have other structures for an endoscope and in which a vent hole is closed by tight contact. For example, it is possible to utilize the feature of the invention for the contact surfaces of the metal sleeve (24) and the metal hat (27) of the air/water supply button (11) according to Figs. 12 and 13 of JP-A 10-028670 (corresponding to JP-B 3017957). In this structure, the contact surfaces are contacted on one another before seal packing on a first one of those is deformed resiliently by a second one thereof. Thus, the vent hole (24g) can be reliably closed by the air-tight contact between the contact surfaces. At the end of the stroke in the down position, there occurs no addition of a deformation amount of the packing to the shift amount of the piston unit. The piston unit can be correctly positioned in the cylinder in both of the halfway position and the down position. It is possible to reduce the predetermined stroke, because a partial component of the stroke for play is not required.

According to the invention, the piston unit can be correctly positioned even in other button assemblies for use in an endoscope. For example, a button assembly may be an air/water supply button assembly. The air-tightness between the contact surfaces can be ensured by resilient deformation of seal packing associated with one of the two contact surfaces. It is possible to prevent unwanted influence of a deformation amount of the seal packing. The piston unit can be set in each of the halfway position and the down position in an expected manner without errors of the deformation. The stroke of the button assembly can be reduced as an initially predetermined value. As a result, the button assembly can have a compact structure with a good manual touch.

In the suction button assembly 29 of the embodiment, three channels including the suction channel 28, the balloon compression channel 35 and the discharge conduit 46 are formed for changeover. Furthermore, the number of the channels formed in the suction button assembly 29 of the invention can be four or more.

In the above embodiment, the endoscope is the ultrasonic endoscope 10. However, an endoscope of the invention with the suction button assembly 29 can be any of various types, for example, colonoscope for entry in a large intestine.

## Claims

1. A switching valve assembly for an endoscope (10), including a cylinder (50), a piston unit (51) disposed in said cylinder in a slidable manner, a button device (43, 47) secured to an upper end of said piston unit, a flow channel (71, 72) formed in said piston unit, plural flow openings (56, 57) formed in said cylinder, wherein said piston unit is set in an initial position at an upper end of said cylinder, a down position, and a halfway position predetermined between said initial position and said down position upon operation of said button device, and changes over plural flow lines (28, 46) between communication and interruption by changing a combination of said flow openings with said flow channel for communication, said switching valve assembly comprising:
a cylinder cap device (76), secured to said cylinder under said button device, for partially covering said piston unit;
a slide device (77) of a cup shape, contained in said cylinder cap device, having a receiving hole (101), for receiving entry of said piston unit, for guiding said piston unit in a longitudinal direction thereof, and for sliding in a downward direction in said cylinder cap device when said button device is pushed in said downward direction;
an end ring (96) for keeping said slide device contained in said cylinder cap device;
a first coil spring (78), disposed between said slide device and said cylinder cap device, for biasing said slide device in an upward direction from said cylinder;
a second coil spring (79), disposed between said button device and said slide device; a first contact surface (107a) disposed on said button device, and opposed to said slide device;
a second contact surface (77b), disposed on said slide device, for contacting said first contact surface when said piston unit is slid to said halfway position by shift of said button device; **characterised in that** the second coil spring is adapted for biasing said button device in said upward direction with smaller force of bias than said first coil spring and **in that** the switching valve assembly further comprises a seal packing (109), associated with one of said first and second contact surfaces, for deforming resiliently to seal said first and second contact surfaces together in an air-tight manner upon contacting one another; and vent hole (103), formed in said slide device through said second contact surface, surrounded by said seal packing, for connection with one of said plural flow lines to come open to atmosphere.

2. A switching valve assembly as defined in claim 1, wherein each of said first and second contact surfaces is constituted by a wall of metal.

3. A switching valve assembly as defined in claim 1 or 2, further comprising an air-tight packing (98), fitted on said slide device, for contacting an inner surface of said cylinder cap device in an air-tight manner.

## Patentansprüche

1. Schaltventilanordnung für ein Endoskop (10), enthaltend einen Zylinder (50), eine in dem Zylinder gleitfähig angeordnete Kolbeneinheit (51), eine an einem oberen Ende der Kolbeneinheit (51) angebrachte Tasteneinrichtung (43, 47), einen in der Kolbeneinheit ausgebildeten Strömungskanal (71, 72), mehrere in dem Zylinder ausgebildete Strömungsöffnungen (56, 57), wobei die Kolbeneinheit in einer Ausgangsstellung an einem oberen Ende des Zylinders, einer Abwärtsstellung und einer Halbwegsstellung zwischen der Ausgangsstellung und der Abwärtsstellung bei Betätigung der Tasteneinrichtung eingestellt wird und mehrere Strömungsleitungen (28, 46) umschaltet zwischen Kommunikation und Unterbrechung durch Ändern einer Kombination der Strömungsöffnungen mit dem Strömungskanal für eine Kommunikation, wobei die Schaltventilanordnung umfasst:
eine Zylinderdeckeleinrichtung (76), die an dem Zylinder unterhalb der Tasteneinrichtung festgelegt ist, um die Kolbeneinheit teilweise abzudecken;
eine in der Zylinderdeckeleinrichtung enthaltene, becherförmige Gleiteinrichtung (77) zum Aufnehmen des Eintritts der Kolbeneinheit, zum Leiten der Kolbeneinheit in deren Längsrichtung, und zum Gleiten in Abwärtsrichtung innerhalb der Zylinderdeckeleinrichtung, wenn die Tasteneinrichtung in Abwärtsrichtung gedrückt wird;
einen Endring (96) zum Halten der in der Zylinderdeckeleinrichtung aufgenommenen Gleiteinrichtung;
eine erste Schraubenfeder (78), angeordnet zwischen der Gleiteinrichtung und der Zylinderdeckeleinrichtung, um die Gleiteinrichtung in Abwärtsrichtung gegenüber dem Zylinder vorzuspannen;
eine zweite Schraubenfeder (79), angeordnet zwischen der Tasteneinrichtung und der Gleiteinrichtung;
eine erste Kontaktfläche (107a), angeordnet an der Tasteneinrichtung gegenüber der Gleiteinrichtung;
eine zweite Kontaktfläche (77b), angeordnet an der Gleiteinrichtung zum Kontaktieren der ersten Kontaktfläche, wenn die Kolbeneinheit durch Verschieben der Tasteneinrichtung in die Halbwegsstellung gleitet;
**dadurch gekennzeichnet, dass** die zweite Schraubenfeder dazu ausgebildet ist, die Tasteneinrichtung in der Aufwärtsrichtung mit geringerer Vorspannkraft vorzuspannen als die erste Schraubenfeder, und dass die Schaltventilanordnung weiterhin eine Dichtungspackung (109) aufweist, die einer von der ersten und der zweiten Kontaktfläche zugeordnet ist, um durch elastische Verformung die erste und die zweite Kontaktfläche miteinander in luftdichter Weise bei deren Kontaktgabe abzudichten; und
ein Entlüftungsloch (103) vorgesehen, das in der Gleiteinrichtung durch die zweite Kontaktfläche hindurch ausgebildet ist, umgeben von der Dichtungspackung zur Verbindung mit einer der mehreren Strömungsleitungen, um diese zur Atmosphäre hin zu öffnen.

2. Schaltventilanordnung nach Anspruch 1, bei der jede von der ersten und der zweiten Kontaktfläche durch eine Metallwand gebildet wird.

3. Schaltventilanordnung nach Anspruch 1 oder 2, weiterhin umfassend eine luftdichte Packung (98), die an der Gleiteinrichtung angepasst ist, um die Innenfläche der Zylinderdeckeleinrichtung luftdicht zu kontaktieren.

## Revendications

1. Ensemble formant soupape de commutation pour endoscope (10),
incluant un cylindre (50), une unité de piston (51) disposée dans ledit cylindre d'une manière permettant le coulissement, un dispositif de bouton (43, 47) fixé sur une extrémité supérieure de ladite unité de piston, un canal d'écoulement (71, 72) formé dans ladite unité de piston, plusieurs ouvertures d'écoulement (56, 57) formées dans ledit cylindre, dans lequel ladite unité de piston est réglée dans une position initiale sur une extrémité supérieure dudit cylindre, une position basse, et une position de mi-course prédéterminée entre ladite position initiale et ladite position basse suite au fonctionnement dudit dispositif de bouton, et fait passer plusieurs lignes d'écoulement (28, 46) entre communication et interruption en modifiant une combinaison desdites ouvertures d'écoulement avec ledit canal d'écoulement pour communication, ledit ensemble formant soupape de commutation comprenant :
un dispositif de couvercle de cylindre (76), fixé audit cylindre sous ledit dispositif de bouton, pour couvrir partiellement ladite unité de piston ;
un dispositif de glissière (77) en forme de coupelle, contenu dans ledit dispositif de couvercle de cylindre, présentant un trou récepteur (101), destiné à recevoir l'entrée de ladite unité de piston, destiné à guider ladite unité de piston dans une direction longitudinale de celle-ci, et destiné à coulisser dans une direction descendante dans ledit dispositif de couvercle de cylindre lorsque ledit dispositif de bouton est poussé dans ladite direction descendante ;
un anneau d'extrémité (96) destiné à maintenir ledit dispositif de glissière contenu dans ledit dispositif de couvercle de cylindre ;
un premier ressort à enroulement (78), disposé entre ledit dispositif de glissière et ledit dispositif de couvercle de cylindre, destiné à solliciter ledit dispositif de glissière dans une direction ascendante à partir dudit cylindre ;
un second ressort à enroulement (79), disposé entre ledit dispositif de bouton et ledit dispositif de glissière,
une première surface de contact (107a) disposée sur ledit dispositif de bouton, et opposée audit dispositif de glissière ;
une seconde surface de contact (77b), disposée sur ledit dispositif de glissière, pour venir en contact avec ladite première surface de contact lorsque ladite unité de piston est coulissée vers ladite position de mi-course par le déplacement dudit dispositif de bouton ;
**caractérisé en ce que** le second ressort à enroulement est apte à solliciter ledit dispositif dans ladite direction ascendante avec une force plus petite que ledit premier ressort à enroulement, et **en ce que** l'ensemble formant soupape de commutation comprend en outre une garniture d'étanchéité (109), associée à une desdites première et seconde surfaces de contact, destinée à une déformation résiliente pour sceller lesdites premières et seconde surfaces de contact ensemble d'une manière étanche à l'air suite à leur mise en contact l'une avec l'autre,
et un trou d'aération (103), formé dans ledit dispositif de glissière à travers ladite seconde surface de contact, entouré par ladite garniture d'étanchéité, pour un raccordement avec une de la pluralité de lignes d'écoulement pour s'ouvrir vers l'atmosphère.

2. Ensemble formant soupape de commutation selon la revendication 1, dans lequel chacune desdites première et seconde surfaces de contact est constituée d'une paroi en métal.

3. Ensemble formant soupape de commutation selon la revendication 1 ou 2, comprenant en outre une garniture étanche à l'air (98), montée sur ledit dispositif de glissière, pour venir en contact avec une surface intérieure dudit dispositif de couvercle de cylindre d'une manière étanche à l'air.
